# EUROPEAN PATENT APPLICATION

(11) **EP 1 918 018 A2**
(43) Date of publication of application: **07.05.2008**
(21) Application number: 08002346.8
(22) Date of filing: 09.11.2004
(51) Int. Cl.: B01D 61/42

(54) **Apparatus for concentrating and purifying nucleic acid**

(30) Priority: 10.11.2003 JP 2003379796
(62) Divisional of application: 04818240.6
(71) Applicant: Arkray, Inc., Kyoto-shi, Kyoto 601-8045 (JP)
(72) Inventor: Hashiguchi, Satoshi, Kyoto-shi Kyoto 601-8045 (JP); Inose, Ken, Kyoto-shi Kyoto 601-8045 (JP)
(74) Representative: Jostarndt, Hans-Dieter

(57) **Abstract**

The conventional method of purifying and concentrating nucleic acids, because of dangerous chemicals, requires elaborate chemical equipment to result in restriction of environment available. Further, time-consuming operation is inevitable and high-speed centrifugation, etc. are needed to cause automation to be difficult and to cause high purification degree to be unattainable. Still further, in the purification method using a column/filter, application of dusty samples tends to invite clogging to lead to a drop of purification efficiency, and centrifugation or suction operation is needed to cause automation to be difficult. In this invention, surfactants (3,4) are adsorbed on impurity (2) contained in a sample, so that the impurity (2) conducts behavior different from that of nucleic acid (1) to thereby attain separation of the impurity (2) from the nucleic acid (1). Impurity (2) other than nucleic acid (1) is energized with cationic surfactant (4) and nonionic surfactant (3) and placed in an electric field to thereby effect separation and purification of the nucleic acid (1) for an analyte containing the impurity (2). Thus, the nucleic acid (1) is brought into the state of being concentrated or easily concentrated.

## Description

### Technical Field

The invention relates to a device for pretreatment of a sample. Particularly, it relates to a pretreatment device for extracting nucleic acids as inspection targets from germs.

### Background Art

Recently, analyses of human genome has been progressed, and various relations between life phenomena and genes. Due to these results, the vision field of medical science and medical service is expanded from pathology to etiology, and from treatment to prevention. Here, the technology of genetic screening forms an important base.

The genetic screening is used for identification of fastidious pathogenic microorganisms, extraction of pathogenic microorganisms from a patient under antibiotic treatment or in a primitive stage of invasion, search of infection source of microorganism, personal identification such as parental relation, diagnosis of genotypic disease types of leukemia and solid tumor, determination of hereditary disease, and so on. These are difficult to have satisfactory result by use of conventional clinical inspections. In comparison with the examinations using culture of microorganism, the genetic screening has a short time required to have a result, thereby being advantageous in examining microorganism requiring a long time to be cultivated. Further, DNA can be extracted from a frozen organism or a stale specimen such as a bone, because DNA is stable under a suitable preservation condition.

Further, the genetic screening attracts people's attention because it has possibility of expanding an inspective field to cover the sexual transmitted diseases on increasing.
Conventionally, there are well-known methods for concentrating and purifying nucleic acid, such as a method using phenol/chloroform/ethanol, a method using a column/filter for adsorbing nucleic acid, and a method using magnetic silica beads.
Further, as disclosed in Japanese Utility Model Laid Open Gazette No. Hei 5-88296, there is a well-known method for collecting nucleic acids from a plate-shaped electrophoresis gel, in which nucleic acids are electrophoretically moved, and subsequently, a collection device is moved to the position of target nucleic acids, and then, the target nucleic acids are further electrophoretically moved so as to be collected by the collection device.

Further, as disclosed in Japanese Utility Model Laid Open Gazette No. Hei 8-327595, there is another well-known method for collecting nucleic acids from a plate-shaped electrophoresis gel, in which target nucleic acids are separated by electrophoresis and a collection chip is inserted into the gel adjacent to a band of target nucleic acids so as to collect the target nucleic acids.

However, the conventional purification method using phenol/chloroform/ethanol requires elaborative chemical equipment for the use of dangerous chemicals, thereby resulting in restriction of environment available. Further, this method is difficult to be automated because it requires time-consuming operation and high-speed centrifugation. Further, this method is difficult to attain high purification level.

In the purification method using a column/filter, application of a dusty sample tends to invite clogging to lead to a drop of purification efficiency, because the method is performed with flow of solution. Further, this method is difficult to be automated because it requires centrifugation or suction operation.

Further, the purification method using magnetic silica beads has a possibility of contamination of a sample with silica when a magnet fails to recover the beads, or when a bead falls from the magnet, thereby being difficult to attain high collection rate.
In the method for collecting nucleic acids from a plate-shaped electrophoresis gel, the plate-shaped electrophoresis gel is needed. Further, this method requires electrophoresis and treatment of a portion of the gel involving target nucleic acids.

The gel used for the electrophoresis has low resistance against shock, and becomes greatly uneven in quality due to its creation process. Therefore, in a generic process, after the electrophoresis is performed, the position of target nucleic acids in the electrophoresis gel has to be recognized by ultraviolet ray before the treatment of a portion of the gel containing many target nucleic acids.
Consequently, if this method is used for the genetic screening or the like, each inspection takes a long time. Further, the gel for electrophoresis is large so as to cause bleeding of the nucleic acid band due to unevenness thereof, thereby being possible to cause low collection rate. Further, large electric power is required for a large gel.

### Disclosure of the Invention

### Problem to Be Solved by the Invention

The problem to be solved is that the methods for concentrating and purifying nucleic acids require complex and elaborate chemical equipment.

### Means for Solving the Problem

To solve the above problem, various examinations have been performed. As a result, it is found that surfactant, which is adsorbed on impurity contained in a sample so as to make the impurity conduct behavior different from that of nucleic acids, is available for separation of the impurity from the nucleic acids.

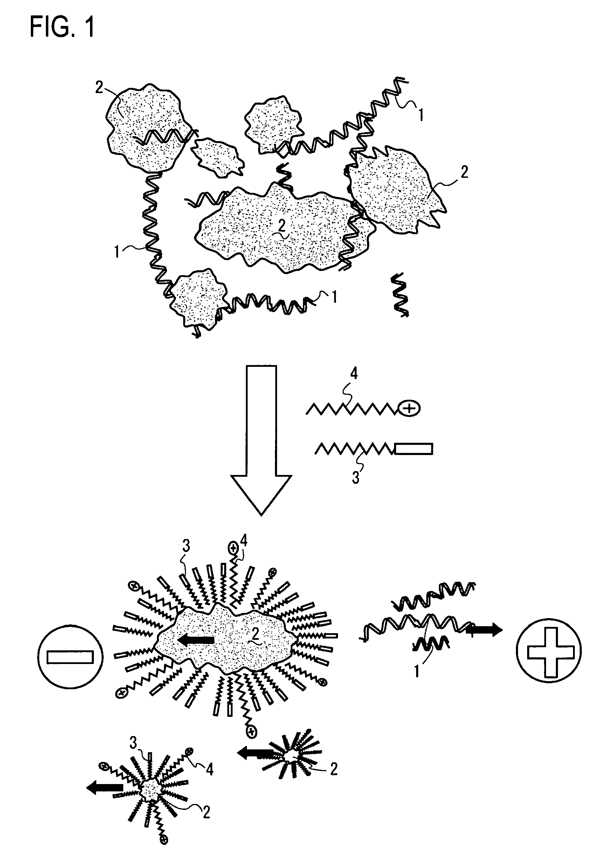

Impurity other than nucleic acids is energized with cationic surfactant and nonionic surfactant and placed in an electric field so as to effect separation and purification of the nucleic acids from a sample containing the impurity. Thus, the nucleic acids are brought into the state of being concentrated or easily concentrated. Fig. 1 is a structural diagram of concentration of nucleic acids by electrophoresis under the existence of surfactant.

A sample contains nucleic acids 1 and impurity 2. Surfactants, that is, nonionic surfactant 3 and cationic surfactant 4, are added into the sample.

The surfactant contained in the sample is adsorbed onto impurity 2. The adsorption of the cationic surfactant onto the nucleic acid can be adjusted by adjusting the electric charge of the nonionic surfactant or by adding salt, e.g., sodium chloride, or polyanionic reagent (heparin, dextran sulfate, DNA).

Impurity 2 on which cationic surfactant 4 is adsorbed is positively energized. The electrophoresis can separate impurity 2 with the surfactant adsorbed thereon from nucleic acids 1 onto which no or little surfactant is adsorbed. Therefore, impurity 2 can be easily removed from the sample so as to effect concentration of nucleic acids 1.
A procedure for concentrating nucleic acids will now be described.

In this procedure, twice electrophoreses are performed for ensuring concentration of nucleic acids. The first electrophoresis removes waste ion from a sample, and the second electrophoresis concentrates nucleic acids in the sample.
Examples of the nonionic surfactant to be added into the sample containing nucleic acids are polyoxyethylene-p-t-octylphenyl ether (such as Triton surfactant, e.g., TritonX-100), polyoxyethylene sorbitan alkyl ester (such as Tween surfactant, e.g., Tween-20), polyoxyethylene alkyl ether (such as Brij surfactant, e.g., Brij35). 1 % TritonX-100 is preferable. If a cell membrane or nuclear membrane is not dissolved, the nonionic surfactant is added and the sample is heated at 96 °C for 10 minutes.

Examples of the cationic surfactant to be added into the sample are zephyramine, cetyl trimethyl ammonium chloride, cetyl pyridinium methyl ammonium chloride, decyl pyridinium chloride (DPC). Addition of 100 µL solution of 0.2 % TritonX-100 is preferable.

Both the nonionic surfactant and the cationic surfactant may be simultaneously added to the sample before heating of the sample.

Even if nucleic acid exits in prokaryotic cell such as Escherichia coli, the surfactant added into the sample in the pretreatment breaks cell membrane of the analyst. In this way, culture solution of Escherichia coli can serve as the sample, and the pretreatment of sample can be easily operated.

After this pretreatment of sample, the sample is energized by direct current with voltage of 100 V for 10 minutes so as to conduct electrophoresis for removing waste ion.

Afterward, the sample is energized by direct current with voltage of 125-150 V for 120 minutes so as to conduct electrophoresis for collecting nucleic acids from an anode. Configuration of an electrophoresis tank for electrophoresis of a sample will be described.

The first electrophoresis will be described.

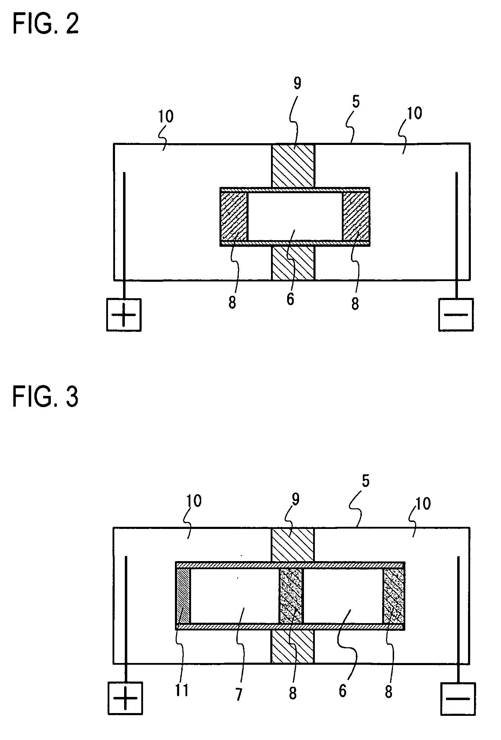

Fig. 2 illustrates configuration of an electrophoresis tank during the first electrophoresis.

Inside an electrophoresis tank 5 are disposed a sample tank 6 and a partition 9 dividing the inner space of tank 5 into an anode side chamber and a cathode side chamber. Sample tank 6 penetrates partition 9 so as to project at one end thereof into the anode side chamber, and at the other end thereof into the cathode side chamber. Both ends of sample tank 6 are opened, and blocked with respective gels 8. Therefore, a potential difference is generated in sample tank 6 so as to cause electrophoresis of nucleic acid and electrophoresis of impurity onto which surfactant is adsorbed. Nonionic surfactant and cationic surfactant are added to a sample, the sample is heated, and then, the sample is introduced into sample tank 6.

Then, the electrodes are energized therebetween with voltage of 100 V is charged between the electrodes so as to conduct electrophoresis for 10 minutes, thereby removing waste ion from the sample before the second electrophoresis.
The second electrophoresis will be described.

The sample tank supplied therein with the sample by the first electrophoresis is connected with a nucleic acid collection tank, and disposed in the electrophoresis tank so as to be subjected to the second electrophoresis.

Fig. 3 illustrates configuration of the electrophoresis tank during the second electrophoresis.

Inside electrophoresis tank 5 are disposed sample tank 6, a collection tank 7 and partition 9 dividing the inner space of tank 5 into an anode side chamber and a cathode side chamber. Sample tank 6 is inserted into partition 9 so as to project into the cathode side chamber. Collection tank 7 is inserted into partition 9 so as to project into the anode side chamber. Sample tank 6 and collection tank 7 are connected to each other through a gel 8 within partition 9.

In this electrophoresis tank, the electrodes are energized therebetween with voltage of 120 V so as to conduct electrophoresis for 120 minutes. The sample, from which waste ion has been already removed, is subjected to the second electrophoresis in the electrophoresis tank, so that nucleic acids can be efficiently collected into collection tank 7. An ultrafiltration membrane 11 is disposed in the anode side chamber so as to prevent nucleic acids from leaking out from collection tank 7, thereby increasing the rate of collection of nucleic acids.

Alternatively, the first electrophoresis may be omitted, i.e., only the second electrophoresis may be performed, if the sample condition is suitable. In this regard, the sample after the pretreatment is supplied into sample tank 6 connected with collection tank 7, and subjected to electrophoresis, thereby easily concentrating nucleic acid. Waste ion contained in the sample is filtrated out through the ultrafiltration membrane so as not to influence the collection of nucleic acids.
According to a first aspect of the invention, in a method for concentrating and purifying nucleic acid by use of electrophoresis, electric charge on impurity contained together with nucleic acid in a sample is adjusted before the sample is placed into an electric field so as to concentrate and purify the nucleic acid.
According to a second aspect of the invention, in a method for concentrating and purifying nucleic acid by use of electrophoresis, cationic surfactant is added into a sample containing nucleic acid so as to adjust electric charge on impurity contained in the sample, and then the sample is placed in an electric field and subjected to electrophoresis so as to concentrate and purify nucleic acid.
According to a third aspect of the invention, in a method for concentrating and purifying nucleic acid by use of electrophoresis, cationic surfactant and nonionic surfactant are added into a sample containing nucleic acid so as to adjust electric charge on impurity contained in the sample, and then the sample is placed in an electric field and subjected to electrophoresis so as to concentrate and purify nucleic acid.
According to a fourth aspect of the invention, the cationic surfactant is adsorbed on the matter other than nucleic acid so as to adjust electric charge on the matter, and the adsorption degree of the cationic surfactant is adjusted by the amount of the added nonionic surfactant.
According to a fifth aspect of the invention, a device is configured so that nonionic surfactant and cationic surfactant are added to a sample, and the sample is subjected to electrophoresis so as to concentrate and purify nucleic acid on an anode side.
According to a sixth aspect of the invention, a device for concentrating and purifying nucleic acid by use of electrophoresis is configured so that a container having side surfaces made of isolative material is partitioned into a sample introduction chamber and a nucleic acid collection chamber by an electro-conductive separation member for prevention of expansion, and the container is connected at an end thereof to an electrode through a buffer tank.

### Effects of the Invention

According to the first aspect of the invention, in a method for concentrating and purifying nucleic acid by use of electrophoresis, since electric charge on impurity contained together with nucleic acid in a sample is adjusted before the sample is placed into an electric field so as to concentrate and purify the nucleic acid, the nucleic acid and the impurity differ in behavior by the electrophoresis so that the nucleic acid can efficiently separated from the impurity. The difference of behavior between the nucleic acid and the impurity can be adjusted due to the electric charge. Thus, the behaviors can be easily controlled.

According to the second aspect of the invention, in a method for concentrating and purifying nucleic acid by use of electrophoresis, cationic surfactant is added into a sample containing nucleic acid so as to adjust electric charge on impurity contained in the sample, and then the sample is placed in an electric field and subjected to electrophoresis so as to concentrate and purify nucleic acid. The addition of surfactant is easily operated so as to easily ensure safety of treatment.

The difference of behavior between the nucleic acid and the impurity in the electrophoresis can be increased by increasing the adsorption amount of the cationic surfactant onto the impurity, so as to effect efficient separation of the nucleic acid. The impurity is electrophoretically moved opposite to the nucleic acid, thereby being prevented from interfering with purification. Thus, the nucleic acid can be easily purified.

According to the third aspect of the invention, in a method for concentrating and purifying nucleic acid by use of electrophoresis, cationic surfactant and nonionic surfactant are added into a sample containing nucleic acid so as to adjust electric charge on impurity contained in the sample, and then the sample is placed in an electric field and subjected to electrophoresis so as to concentrate and purify nucleic acid. Therefore, the adsorption amount of the cationic surfactant is adjusted due to the ratio between the cationic surfactant and the nonionic surfactant. Thus, the behavior of the impurity in the electrophoresis can be easily controlled.

Additionally, due to adsorption of the nonionic surfactant onto the nucleic acid, the cationic surfactant can be prevented from being adsorbed on the nucleic acid.

According to the fourth aspect of the invention, the cationic surfactant is adsorbed on the matter other than nucleic acid so as to adjust electric charge on the matter, and the adsorption degree of the cationic surfactant is adjusted by the amount of the added nonionic surfactant. Therefore, the ratio between the cationic surfactant and the nonionic surfactant about adsorption onto the impurity can be controlled by such an easy operation. Further, electric charge on the foreign mater can be adjusted by such an easy operation. Additionally, due to adsorption of the nonionic surfactant onto the nucleic acid, the cationic surfactant can be prevented from being adsorbed on the nucleic acid.

According to the fifth aspect of the invention, a device is configured so that nonionic surfactant and cationic surfactant are added to a sample, and the sample is subjected to electrophoresis so as to concentrate and purify nucleic acid on an anode side. Such a simple device can purify nucleic acid in a sample. Further, the concentrating and purifying device can be minimized while ensuring safety because the device can control the behavior of nucleic acid.

According to the sixth aspect of the invention, a device for concentrating and purifying nucleic acid by use of electrophoresis is configured so that a container having side surfaces made of isolative material is partitioned into a sample introduction chamber and a nucleic acid collection chamber by an electro-conductive separation member for prevention of expansion, and the container is connected at an end thereof to an electrode through a buffer tank. Therefore, the concentrating and purifying device is simplified so as to save manufacturing costs. Further, the device is easily controllable and greatly safe.

### Brief Description of the Drawings

Fig. 1 is a structural diagram of concentration of nucleic acids by electrophoresis with existence of surfactants.
Fig. 2 is a structural diagram of an electrophoresis tank for first electrophoresis.
Fig. 3 is a structural diagram of an electrophoresis tank for second electrophoresis.
Fig. 4 is a structural diagram of a first electrophoresis tank.
Fig. 5 is a structural diagram of a second electrophoresis tank.
Fig. 6 is a perspective view of a sampling unit.
Fig. 7 is a plan view of the sampling unit.
Fig. 8 is a side view of the sampling unit.
Fig. 9 is a sectional side view of the sampling unit.
Fig. 10 is a perspective view of a connection part.
Fig. 11 is a sectional side view of the connection part.
Fig. 12 is a sectional side view of a filtration part.
Fig. 13 is a view of a separation unit while being assembled.
Fig. 14 is a graph of UV spectrum of collected liquid.
Fig. 15 is a structural diagram of an entire electrophoresis device.
Fig. 16 is a perspective view of an electrophoresis unit.
Fig. 17 is a sectional side view of the electrophoresis unit.
Fig. 18 illustrates diagrams of the electrophoresis unit at the former period of purification.
Fig. 19 illustrates diagrams of the electrophoresis unit at the latter period of purification.
Fig. 20 is a sectional side view of the electrophoresis unit while being assembled.
Fig. 21 is a perspective view of the electrophoresis unit while being assembled.
Fig. 22 illustrates a structure of a first block.
Fig. 23 illustrates a structure of a second block.
Fig. 24 is a front view of a gasket.
Fig. 25 is a graph showing a comparison result of purification of Neisseria gonorrhoeae genome.
Fig. 26 is a view of a gel containing samples showing a result of DNA concentration due to electrophoresis.

### Description of Notations

1 Nucleic Acid
2 Impurity
3 Nonionic Surfactant
4 Cationic Surfactant
5 Electrophoresis Tank
6 Sample Tank
9 Partition

### Best Mode for Carrying out the Invention

An object is to provide a nucleic acid concentration device having high purification rate without using dangerous chemical, and the object is achieved by using surfactant and electrophoresis.

### Embodiment 1

An embodiment of the present invention will be described.

First, configuration of an electrophoresis tank used for electrophoresis will be described.

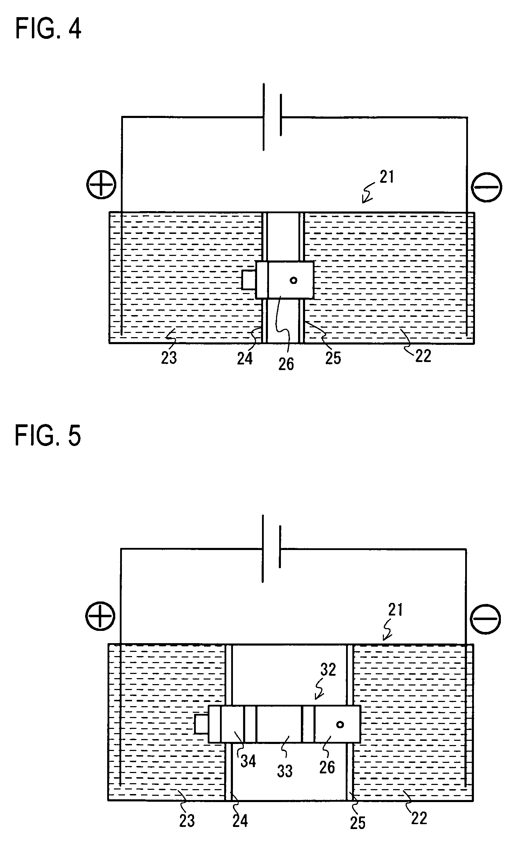

Fig. 4 is a structural diagram of a first electrophoresis tank.

An electrophoresis tank 21 is divided into a cathode side tank 22 and an anode side tank 23 by partitions 24 and 25. Partitions 24 and 25 are disposed at a center portion of electrophoresis tank 21, and a sample unit 26 is fitted in partitions 24 and 25. Sample unit 26 projects at one end thereof into cathode side tank 22, and at the other end thereof into anode side tank 23. Sample unit 26 is blocked with gel on the cathode side thereof, and provided at a side surface thereof with an introduction hole for introducing a sample thereinto. The introduction hole is plugged during electrophoresis.

A cathode is inserted into cathode side tank 22, and an anode is inserted into anode side tank 23, so as to energize electrophoresis tank 21. Another configuration of an electrophoresis tank will be described.

Fig. 5 is a structural diagram of a second electrophoresis tank.

For the second electrophoresis, an electrophoresis tank 21 is divided into a cathode side tank 22 and an anode side tank 23 by partitions 24 and 25. Partitions 24 and 25 are disposed at a center portion of electrophoresis tank 21, and a separation unit 32 is fitted in partitions 24 and 25. Separation unit 32 projects at one end thereof into cathode sided tank 22, and at the other end thereof into anode side tank 23. A cathode is inserted into cathode side tank 22, and an anode is inserted into anode side tank 23, so as to energize electrophoresis tank 21.

Separation unit 32 consists of mutually joined three parts, i.e., sampling unit 26, a connection part 33 and a filtration part 34. O-rings are interposed between sampling unit 26 and connection part 33, and between connection part 33 and filtration part 34, respectively, so as to ensure their joint, and prevent buffer liquid from leaking.

Sampling unit 26 is blocked at the cathode and anode sides thereof with gels, respectively. Filtration part 34 is provided with an ultrafiltration membrane. Such electrophoresis tanks are used for concentrating nucleic acid.

First, for the first electrophoresis, the pretreated sample is poured into the introduction hole, and the introduction hole is plugged. Sample unit 26 is disposed in electrophoresis tank 21 so as to be slightly exposed at the upper surface thereof above liquid. Then, direct current is charged with voltage of 100 V so as to conduct electrophoresis for 20 minutes, thereby removing waste ion from the sample. The buffer liquid is prepared in pH 8.0 by 1xTAE solution, 40mM Tris, 40mM glacial acetic acid and 1mM EDTA.
After the waste ion is removed from first electrophoresis tank 21, connection part 33 and filtration part 34 are connected to sample unit 26. O-rings are provided at respective joint portions so as to prevent leak of the liquid.

Connection part 33 is supplied therein with mixture liquid of 100 % ethanol and 1xTAE, in which the ratio of 100 % ethanol to 1xTAE is 6:4. Sampling unit 26 is supplied therein with TE-1 (10mM Tris-HCL, 0.1mM EDTA, pH 8.0). Filtration part 34 is supplied therein with TE-1. Configurations of sampling unit 26, connection part 33 and filtration part 34 will be described.

First, configuration of sampling unit 26 will be described.

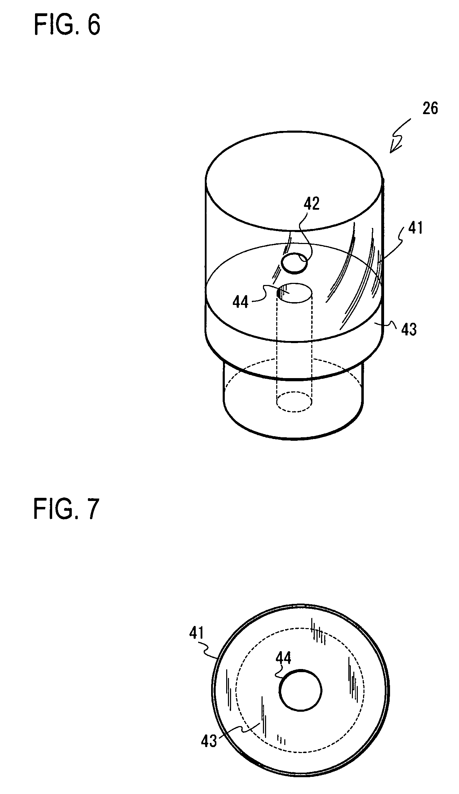

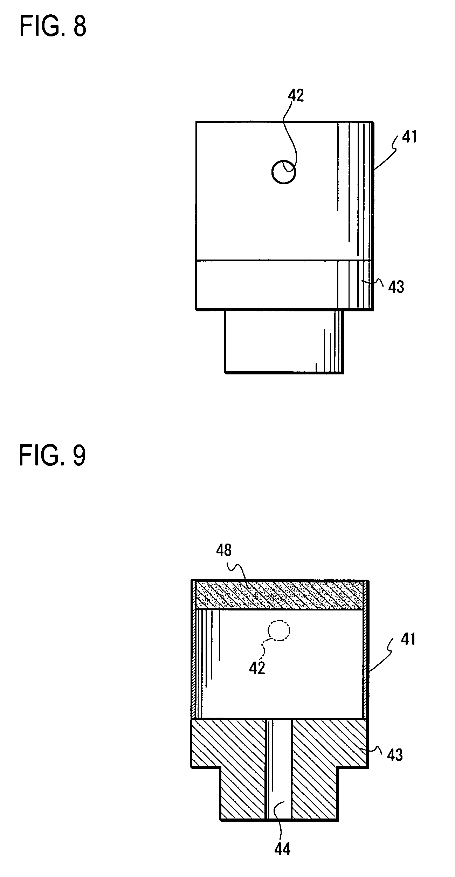

Fig. 6 is a perspective view of a sampling unit; Fig. 7 is a plan view of the sampling unit; Fig. 8 is a side view of the sampling unit; and Fig. 9 is a sectional side view of the sampling unit.

Sampling unit 26 is a container holding gel therein. A centrifugal filter made by Millipore, Microcon (trade mark) YM-3, is treated so that an existing inner ultrafiltration membrane is removed therefrom, and that a new hole having a diameter of 5 mm is bored therein, thereby serving as sampling unit 26. Any member can serve as sampling unit 26 if it has the same effect.

Sampling unit 26 includes a cylindrical body 41 and a base 43. Cylindrical body 41 is connected to base 43 and is bored with an introduction hole 42 for introducing a sample. Base 43 is a stepped circular column with a hole 44 opened at top and bottom surfaces thereof.

Gel 48, having a thickness of several millimeters, is disposed inside cylindrical body 41 so as to block the opening of cylindrical body 41. Therefore, a sample supplied from introduction hole 42 is supplied into the inside of gel 48. Configuration of connection part 33 will be described.

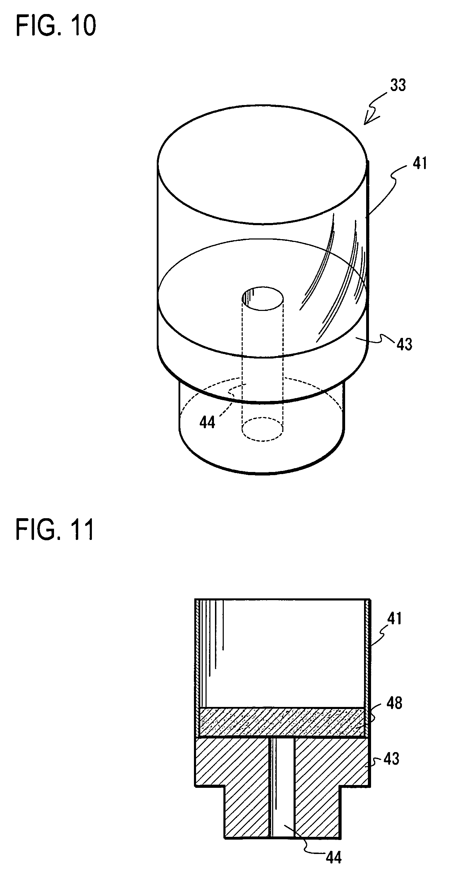

Fig. 10 is a perspective view of the connection part; and Fig. 11 is a sectional side view of the connection part.

The centrifugal filter made by Millipore is also treated so that an existing inner ultrafiltration membrane is removed therefrom, and that a new hole having a diameter of 5 mm is bored therein, thereby serving as connection part 33.

Connection part 33 includes a cylindrical body 41 and a base 43. Cylindrical body 41 is connected to base 43. Base 43 is a stepped circular column with a hole 44 opened at top and bottom surfaces thereof.

Gel 48, having a thickness of several millimeters, is disposed on a top surface of base 43 in cylindrical body 41 so as to prevent liquid from flowing between connection part 33 and sampling unit 26. Configuration of filtration part 34 will be described.

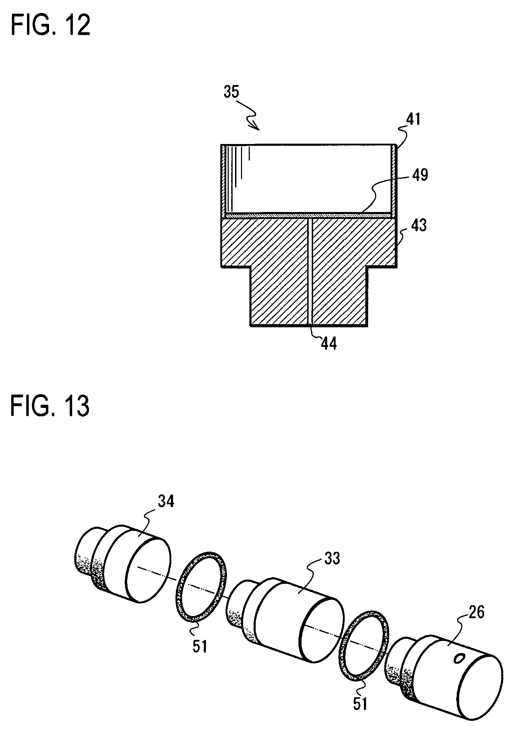

Fig. 12 is a sectional side view of the filtration part.

The centrifugal filter made by Millipore is also treated so as to serve as filtration part 34.

Filtration part 33 includes a cylindrical body 41 and a base 43. Cylindrical body 41 is connected to a base 43. The length of cylindrical body 41 is as large as that of each of sampling unit 26 and connection part 33 minus about 5 mm. Base 43 is a stepped circular column with a hole 44 opened at top and bottom surfaces thereof.

An ultrafiltration membrane 49 is disposed on a top surface of base 43 in cylindrical body 41 so as to prevent leak of nucleic acid, thereby ensuring concentration of nucleic acid.
An example of operation for concentrating nucleic acids will be described.

Culture solution of Escherichia coli serves as a sample, which is subjected to the above-mentioned first and second electrophoreses so as to concentrate nucleic acids. The concentration of collected nucleic acids is measured by an absorbance measurement.

The sample is 100 µL culture solution of Escherichia coli DH5α.

100 µL solution of 1 % Triton (trade mark) X-100 is added to the sample, and heated at a temperature of 96 °C for 10 minutes.

Afterward, 100 µL solution of 0.2 % DPC is added so as to prepare a sample to be subjected to the electrophoreses.
0.5xTAE serves as buffer for the electrophoreses.

1xTAE is used for dissolving agarose.

The 1xTAE solution is prepared in pH 8.0 with 40mM Tris, 40mM glacial acetic acid.

Connection part 33 is left at rest so that the opening side of cylindrical body 41 faces upward. 1 % agarose gel (SeaKem Gold agarose: TaKaRa) is poured into cylindrical body 41 from the opening side so as to accumulate to a thickness of 3-7 mm, and hardened.

Similar to connection part 33, sampling unit 26 is left at rest so that the opening side of cylindrical body 41 faces upward. 1 % agarose gel (SeaKem Gold agarose: TaKaRa) is supplied into cylindrical body 41 from the open side so as to accumulate to a thickness of several millimeters. After the gel becomes hard, sampling unit 26 is vertically reversed so as to pour the gel hardened at the opening side into cylindrical body 41.
HU-6 (made by AR Brown) serves as the electrophoresis tank. MPSU-200 (made by AR Brown) serves as a power source.

The prepared sample to be subjected to electrophoreses is introduced into sampling unit 26 through introduction hole 42, and introduction hole 42 is plugged.

The electrophoresis tank is provided with putty so as to be divided into an anode side chamber and a cathode side chamber. 0.5xTAE is supplied into each of the anode and cathode side chambers.

Sampling unit 26 is fitted in the putty so as to be slightly exposed at an upper surface thereof above the buffer liquid.

Then, direct current is charged with voltage of 100V so as to conduct the first electrophoresis for 20 minutes. Subsequently, connection part 33 and filtration part 34 are connected to sampling unit 26 after the first electrophoresis, so as to constitute the separation unit to be subjected to the second electrophoresis.

An example of operation for the second electrophoresis will be described.

Mixture solution of 100 % ethanol and 1xTAE, in which the rate of 100 % ethanol to 1xTAE is 6:4, is supplied in connection part 33.

TE-1 (10mM Tris-HCL, 0.1mM EDTA, pH 8.0) solution is supplied infiltration part 34.

Then, connection part 33 and filtration part 34 are connected to sampling unit 26, thereby assembling the separation unit.
Fig. 13 is a view of a separation unit while being assembled.

To assemble the separation unit, sampling unit 26, connection part 33 and filtration part 34 are aligned in the same direction, and O-rings 51 are interposed between connection part 33 and filtration part 34, and between connection part 33 and filtration part 34, respectively, so as to prevent leak of liquid from the separation unit.
The electrophoresis tank is provided with putty so as to be divided into an anode chamber side and a cathode side chamber, and 0.5xTAE is supplied into each of the anode and cathode side chambers.

The assembled separation unit is disposed in the putty so as to place the end of sampling unit 26 side on the cathode side, and place filtration part 34 on the anode side.

Then, direct current is charged with voltage of 200 V so as to conduct the second electrophoresis for 240 minutes.
Subsequently, nucleic acid solution is collected from filtration part 34 and the absorbance thereof is measured, so that the concentration of the collected nucleic acids is calculated based on UV spectrum.

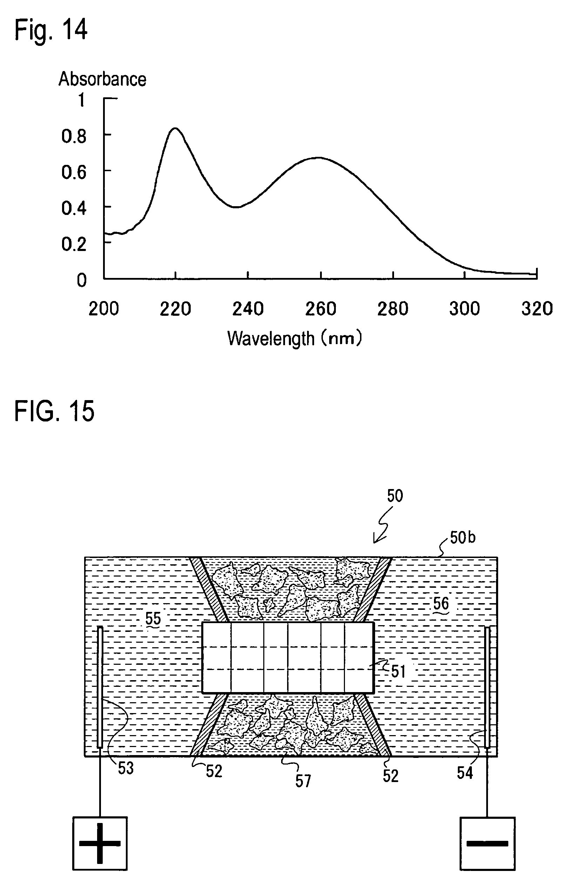

Fig. 14 is a graph of UV spectrum of collected liquid.

The calculated concentration of nucleic acid is 32.3 ng (6.7*106 copy/µL).

The concentration of nucleic acids is calculated so that the absorbance in 260 nm (A260) is multiplied by proper coefficient of the nucleic acid property, and by an optical length of the cell (mm), and divided by 10.
Also, the purity of the collected nucleic acid is calculated based on the UV spectrum.

The calculated purity of nucleic acid is 1.91.

The purity is calculated so that the absorbance in 260 nm (A260) is divided by the absorbance in 280 nm (A280). If the sample is 100% DNA, the calculated value becomes about 1.8. If the sample is 100% RNA, the calculated value becomes 2.0. The value of A280 is a reflex of the amount of protein and phenol contained in the measurement target. If the absorbance ratio is greatly less than 1.5, it should be considered that the sample is contaminated with monomeric substance such as protein.

### Embodiment 2

An electrophoresis device according to a second embodiment will now be described.

Fig. 15 is a structural diagram of an entire electrophoresis device.

An electrophoresis device 50 comprises an electrophoresis tank 50b in which an electrophoresis unit 51 is disposed so as to concentrate nucleic acids contained in a sample. Electrophoresis tank 50b includes buffer tanks 55 and 58 and a cooling water tank 57. Buffer tanks 55 and 56 are filled therein with buffer liquid for electrophoresis. Cooling water tank 57 is filled therein with cooling water, such as iced water, for cooling electrophoresis unit 51. An anode 53 is disposed in buffer tank 55, and a cathode 54 is disposed in buffer tank 56. Electrophoresis unit 51 projects at opposite ends thereof into respective buffer tanks 55 and 56. A sample is introduced into electrophoresis unit 51, and anode 53 and cathode 54 are energized therebetween so as to concentrate nucleic acids in the sample.

Partitions 52 separate cooling water tank 57 from buffer tanks 55 and 56. Partitions 52 and electrophoresis tank 50b are made of isolative material. Partitions 52 may be made of putty such as silicone bulking agent or epoxy resin. Electrophoresis unit 51 is cooled at both sides thereof by the cooling water through partitions 52 so as to cancel heat generation during the electrophoresis, thereby ensure purification of a sample at a stable temperature environment. Electrophoresis unit 51 will now be described.

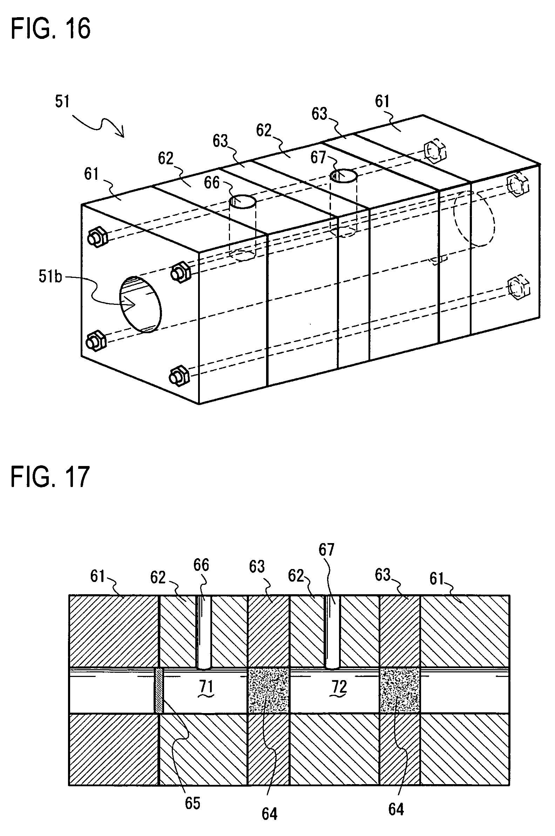

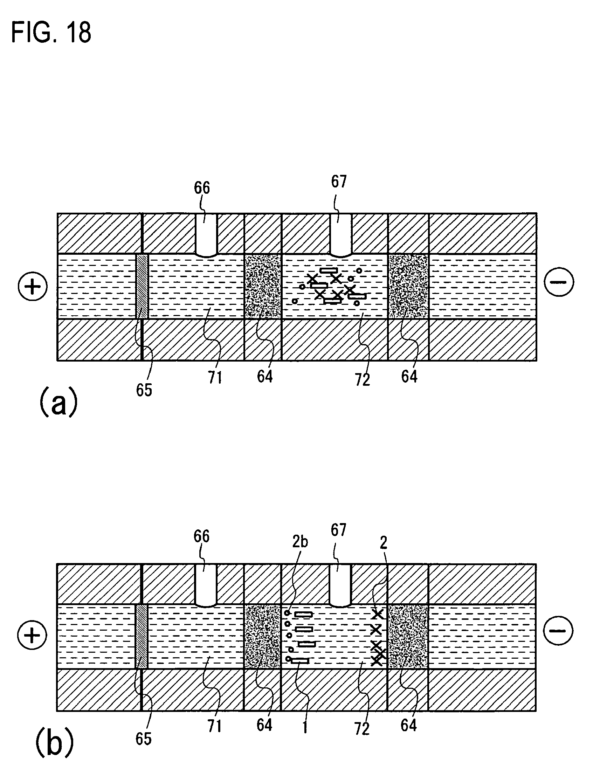

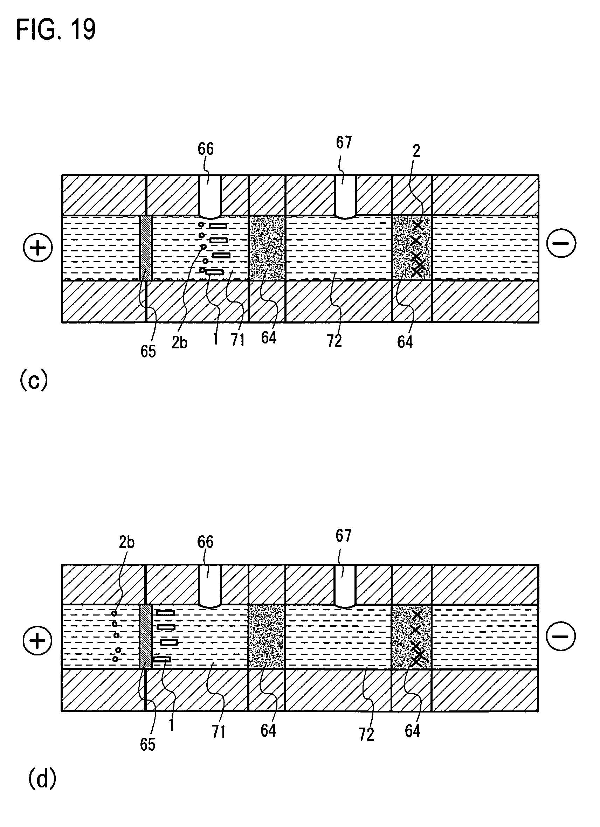

Fig. 16 is a perspective view of an electrophoresis unit; Fig. 17 is a sectional side view of the electrophoresis unit; Fig. 18 illustrates diagrams of the electrophoresis unit at the former period of purification; and Fig. 19 illustrates diagrams of the electrophoresis unit at the latter period of purification.

Electrophoresis unit 51 is mainly made of isolative material such as acrylic resin. Plural members are fastened together with bolts so as to constitute a single body of electrophoresis unit 51. Electrophoresis unit 51 is provided therein with a columnar space 51b along the longitudinal direction thereof, and provided with two holes disposed perpendicular to the longitudinal direction of space 51b. One hole is an introduction hole 67 for introducing a sample into space 51b, and the other is an extraction hole 66 through which the concentrated sample is collected from space 51b. Introduction hole 67 and extraction hole 66 are opened to space 51b.

A collection tank 71 and a sample tank 72 are disposed inside electrophoresis unit 51, so that introduction hole 67 is connected to collection tank 71, and sampling hole 66 is connected to sample tank 72. A gel wall 64 is disposed between sample tank 72 and collection tank 71. Another gel wall 64 is also disposed on the cathode side of sample tank 72. An ultrafiltration membrane 65 is disposed on the anode side of collection tank 71. Namely, sample tank 72 is formed between two gel walls 64, and collection tank 71 is formed between gel wall 64 and ultrafiltration membrane 65.
A purification process by electrophoresis unit 51 will be described.

To purify a sample, space 51b in electrophoresis unit 51 is full of buffer for electrophoresis. Sample tank 72 and collection tank 71 are also full of buffer. As shown in Fig. 18(a), after the sample is introduced into sample tank 72, electrophoresis unit 51 is energized between opposite ends thereof. Here, the sample contains target nucleic acids, impurity, and nucleic acids which are smaller than the target nucleic acids.

When electrophoresis unit 51 is energized, as shown in Fig. 18(b), nucleic acids 1 and waste electrolyte 2b move toward the anode, and impurity 2 moves toward the cathode. By the energization for a certain period, as shown in Fig. 19(c), nucleic acids 1 and waste electrolytes 2b reach collection tank 71 through gel wall 64. By further energization, as shown in Fig. 19(d), waste electrolytes 2b having small molecular weight pass ultrafiltration membrane 65 so as to be exhausted from collection tank 71. Target nucleic acids 1 remain in collection tank 71.

In this way, in electrophoresis unit 51, target nucleic acids 1 can be easily separated from the sample by use of the electrophoresis and the ultrafiltration membrane. Individual parts in electrophoresis units 51 will be described.

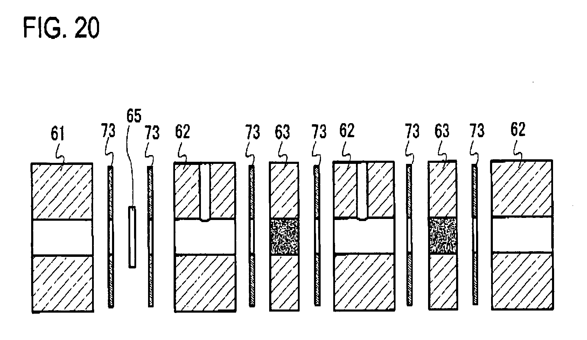

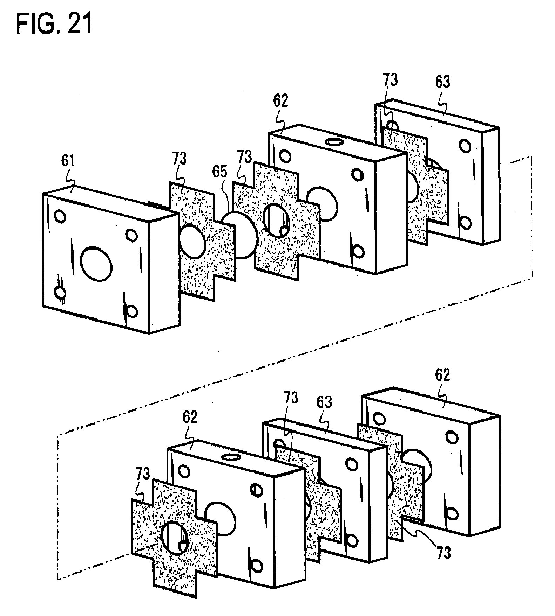

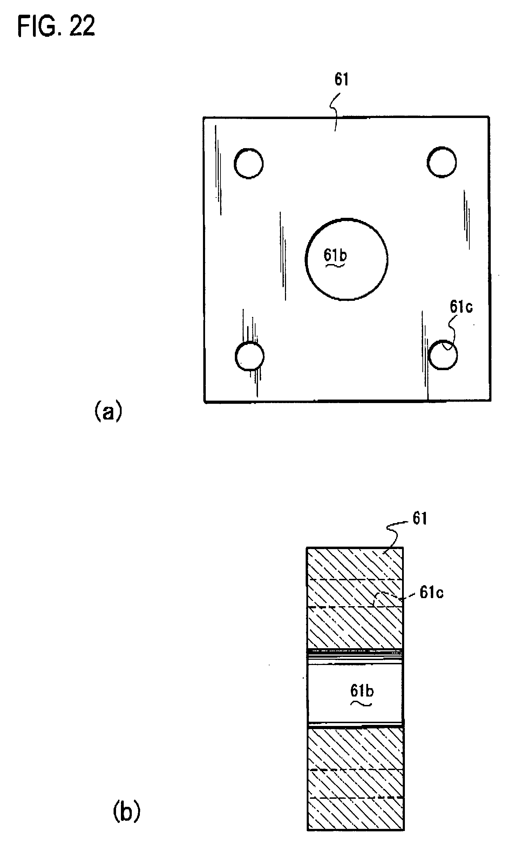

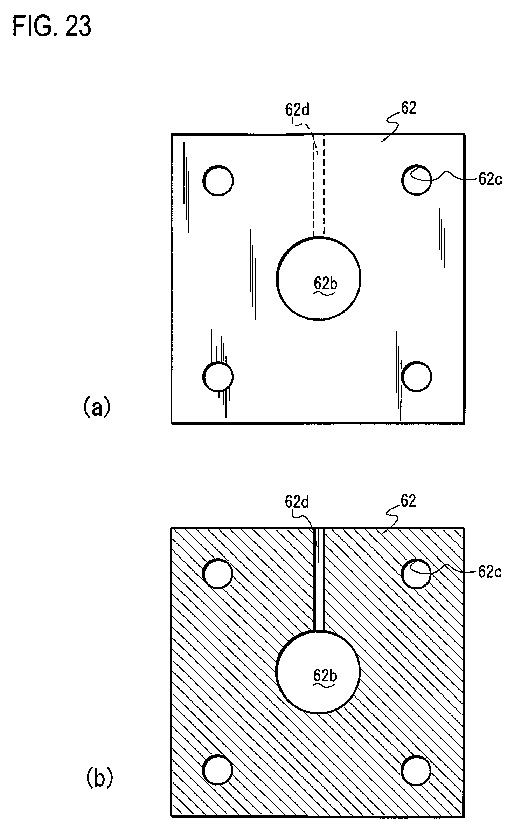

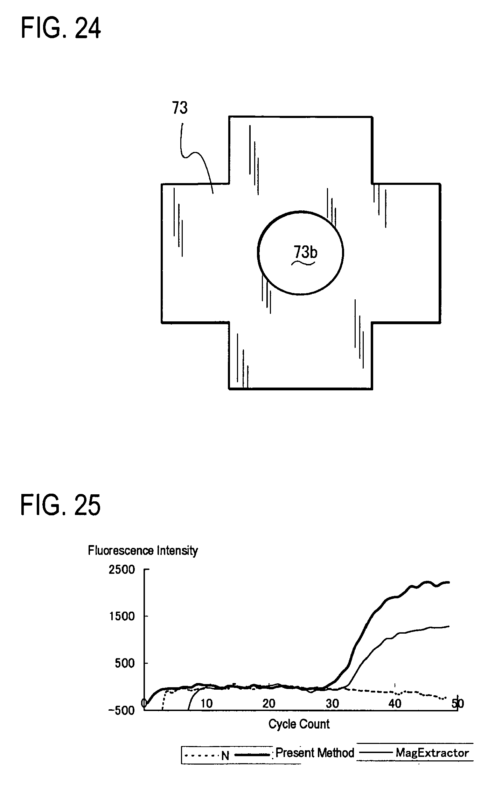

Fig. 20 is a sectional side view of the electrophoresis unit while being assembled; Fig. 21 is a perspective view of the electrophoresis unit while being assembled; Fig. 22 illustrates a structure of a first block; Fig. 23 illustrates a structure of a second block; and Fig. 24 is a front view of a gasket.

Electrophoresis unit 51 is an assemble comprising a first block 61, second blocks 62, third blocks 63, gaskets 73 and an ultrafiltration membrane 65. Each of first block 61, second blocks 62 and third blocks 63 are bored at the axial center portion thereof with a hole opened at front and rear surfaces thereof so as to form a space 51b of electrophoresis unit 51, and each of blocks 61, 62 and 63 is bored with at four corners with respective holes opened at front and rear surfaces thereof so as to be fastened to another block by bolts.

First block 61 serves as an end portion of electrophoresis unit 51. Second blocks 62 serve as sample unit 72 and collection unit 71. Each of third blocks 63 holds gel wall 64. Each of gaskets 73 is disposed between each neighboring pair of the blocks so as to prevent cooling water from entering the inside of electrophoresis unit 51. Ultrafiltration membrane 65 is sandwiched between gaskets 73 and 73 at one end portion of electrophoresis unit 51.
As shown in Fig. 22, first block 61 is bored at the axial center portion thereof with a hole 61b serving as a part of space 51b of electrophoresis unit 51. Further, first block 61 is bored at four corners thereof with holes 61c into which respective bolts are inserted.

As shown in Fig. 23, second block 62 is bored at the axial center portion thereof with a hole 62b serving as a part of space 51b of electrophoresis unit 51. Second block 62 is also bored at four corners thereof with holes 62c into which respective bolts are inserted. Further, second block 62 is bored with a vertical hole 62d opened to hole 62b. Hole 62d serves as either introduction hole 67 or extraction hole 66 of electrophoresis unit 51.

Third block 63 is equivalent to first block 61 thinned in the fore-and-aft direction. Third block 63 is provided at the axial center portion thereof with a hole for holding gel wall 64 therein.

Gasket 73 is a cruciform sheet when viewed in front, made of silicone coat having a thickness of 0.5 mm in this embodiment, and provided at the center portion thereof with a hole 73b. As shown in Fig. 24, gasket 73 is cut off at four corners thereof so as to allow passing of respective bolts for fastening the blocks. Hole 73b is diametrically as large as each of hole 61b of first block 61 and holes 62b of second blocks 62.

Ultrafiltration membrane 65 is larger than hole 73b, so as to be sandwiched between gaskets 73 and 73.
A test of concentrating, purifying and separating nucleic acids by means of the electrophoresis device according to the invention will be described.

### Comparison Test 1

A comparison test 1 is comparison between purification of Neisseria gonorrhoeae genome from cultured urine contaminated with Neisseria gonorrhoeae by using the present electrophoresis device and purification of the same by the method using magnetic silica beads.

The operation using the electrophoresis device of the present embodiment will be described.

Neisseria gonorrhoeae is cultured in chocolate culture medium EX (made by Nissui Pharmaceutical Co., Ltd.) at a temperature of 37 °C for 2 days.

The obtained colony is suspended in physiological saline so as to have an absorbance of OD530=0.18, and then, diluted by physiological saline so as to be a 1/100 solution. In this way, a diluted germ solution is obtained.

The 1.2 µL diluted germ solution is added to 60 µL mixed urine of a healthy person. Further, 60 µL liquefied buffer A, having composition as indicated in Table 1, is added to the urine, so as to provide a mixture solution.

The mixture solution is heated at a temperature of 96°C for 10 minutes. A 100 µL part of the mixture solution serves as a sample, which is introduced into the present electrophoresis device and subjected to electrophoresis for 60 minutes by a voltage of 150 V.

A 5 µL part of the collected 100 µL sample is subjected to polymerase chain reaction (PCR), and its fluorescence intensity is measured.

The prescription of the PCR treatment is indicated in Table 2.

Here, the size of first block 61 is determined to have a width of 20 mm, a height of 20 mm and a thickness of 5 mm, with hole 61b having a diameter of 5 mm. Second block 62 is determined in size similar to first block 61, and further, hole 62d has a diameter of 2 mm. Third block 63 has the same width and height as those of first block 61, and has a thickness of 2.5 mm. Agarose gels serve as the gel walls. SeaKem Gold agarose (made by TaKaRa) is used as the agarose. 10mM Tris-HCl (pH 8.0) serves as the solution liquid. YM-100 (made by Millipore) serves as the ultrafiltration membrane. Gasket 73 has a thickness of 0.5 mm.
The operation using magnetic silica beads will be described.

MagExtractor (made by Toyobo) serves as the magnetic silica beads.

The same operation as the above-mentioned operation using the electrophoresis is performed till the mixture liquid is heated at a temperature of 96 °C for 10 minutes. A 100 µL part of the mixture liquid is extracted as a sample, and treated according to the protocol of MagExtractor. A 5 µL part of the collected 100 µL sample is subjected to PCR treatment, and its fluorescence intensity is measured.

With respect to the heating cycles of the PCR treatment, as indicated in Table 3, retention at a temperature of 50°C for 2 minutes is performed, and then retention at a temperature of 95 °C for 2 minutes is performed, and subsequently, fifty cycles, each of which is combination of retention at a temperature of 95°C for 10 seconds and retention at a temperature of 56°C for 60 seconds, are performed.

**Table 1**

| Composition of Liquefied Buffer A | |
|---|---|
| 2 % (weight) | Triton X-100 |
| 0.1% | Cetyl Tri-Methyl Ammonium Chloride (CTAC) |
| 10mM | Tris-HCl (pH8.0) |
| 10mM | EDTA (pH8.0) |
| 300mM | NaCl |
| 16.6 copy/µL | Human Genome |

**Table 2**

| Prescription of PCR | |
|---|---|
| Prescription for One Reaction | |
| H₂O | 15.15 µL |
| 10X Gene Taq Buffer | 2.5 µL |
| 10mM AUGC | 0.5 µL |
| 100mM MgCl2 | 0.375 µL |
| 5µM F-D-NG-R1-32 | 1 µL |
| 100µM NG-F3405-20 | 0.125 µL |
| 100µM NG 3526-20R | 0.125 µL |
| 2U/µL UNG | 0.1 µL |
| 5U/µL Gene Taq NT | 0.125 µL |
| 20 µL + 5µL Recovered Sample | |

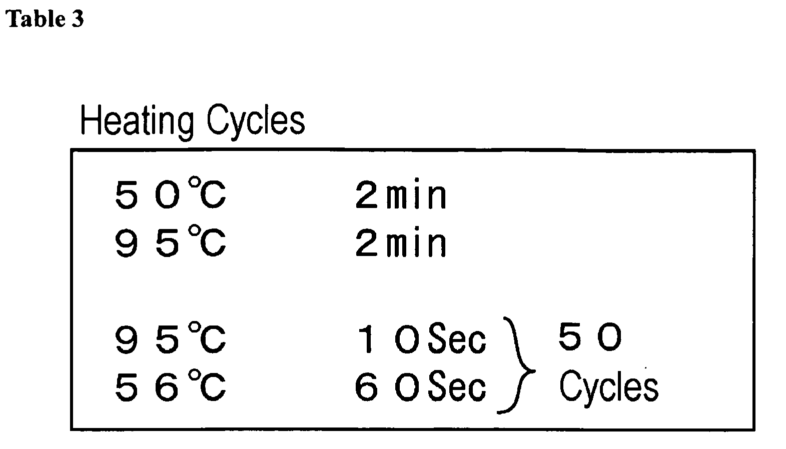

Fig. 25 is a graph showing a comparison result of purification of Neisseria gonorrhoeae genome. In Fig. 25, the wide line represents the purification result of the present method, and the narrow line represents the purification result of the magnetic silica beads method. The dotted line represents the result of negative control.

Consequently, detection of nucleic acid purified by the present method is earlier than that by the magnet silica beads method. This result means that the efficiency of purification and separation of nucleic acid by the present method is higher than that by the magnetic silica beads method.

### Concentration Test

An operation for concentrating DNA by use of the present electrophoresis device will now be described.

In the electrophoresis device of the present embodiment, the capacity of the sample tank is 200 µL, and the capacity of the collection tank is 50 µL.

100 µL λ/HindIII DNA (2.7 ng/µL) is mixed with 100 µL liquefied buffer A so as to prepare a sample. The sample is introduced into the sample tank, and subjected to electrophoresis with a voltage of 100 V for 30 minutes so as to be concentrated.

Subsequently, 2 µL, 4 µL, 6 µL and 8 µL samples are extracted from either of the sample before concentration and the sample after concentration, and subjected to gel electrophoresis. Further, a 10 µL sample is extracted from the collected sample after concentration so as to be subjected to the electrophoresis.

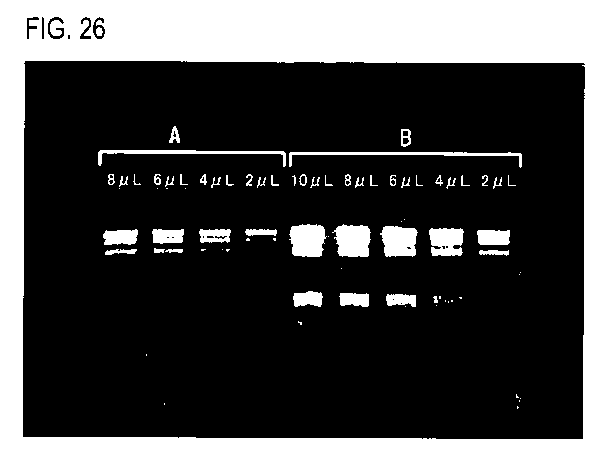

Fig. 26 is a view of a gel containing samples showing a result of DNA concentration due to electrophoresis.

In Fig. 26, the samples before concentration belong to a group A, and the samples after concentration belong to a group B. As shown in Fig. 26, in comparison between the samples having the same amount, it is noticed that a band of the sample after concentration is clearer than a band of the sample before concentration.

In Fig. 26, the band state of the 2 µL collected sample matches the band state of the 6 µL sample before concentration.

In this way, the electrophoresis device of the present embodiment effects concentration of DNA.

### Industrial Applicability

The present invention, whose operability or device structure is simple, is suitable to various applications such as an inspection device for automatically concentrating and inspecting nucleic acids.

## Claims

1. An apparatus for performing electrophoresis for concentration and purification of a nucleic acid, **characterized in that** a container (32, 51), having a side wall formed of an insulator, is divided into a sample introduction chamber (6, 26, 72) and a nucleic acid recovery chamber (7, 34, 71) by a conductive separation medium (8, 48, 64) for preventing diffusion, and that the container (5, 32, 51) has ends connected to respective electrodes (53, 54) through respective buffer tanks (10, 22, 23, 55, 56),

2. The apparatus as set forth in claim 1, wherein the container (32) is configured by combining a first unit (26) having the sample introduction chamber and the separation medium (48) with a second unit (34) having the nucleic acid recovery Chamber.

3. The apparatus as set forth in claim 1, wherein each of the first and second units (26, 34) is configured by connecting a cylindrical body (41) to a base (43) having a penetrating hole (44) opened to the cylindrical body (41), and wherein the first unit (26) including the separation medium (48) blocking an opening of the cylindrical body (41).

4. The apparatus as set forth in claim 3, wherein the container (32) includes a third unit (33) for connecting the sample introduction chamber to the nucleic acid recovery chamber, wherein the third unit (33) is interposed between the first and second units (26, 34), and wherein the third unit (33) is configured by connecting a cylindrical body (41) to a base (43) having a penetrating hole (44) opened to the cylindrical body (41), and is provided with a separation medium (48) blocking an opening of the cylindrical body (41).

5. The apparatus as set forth in claim 1, wherein the container (51) includes a first block (62) having the sample introduction chamber (72), a second block (62) having the nucleic acid recovery chamber (71), and a third block (63) having the separation medium (64), the third block (63) being interposed between the first and second blocks (62).

6. The apparatus as set forth in claim 5, wherein the first, second and third blocks (62,63) are fastened together by a bold.
